# EUROPEAN PATENT APPLICATION

(11) **EP 4 732 768 A1**
(43) Date of publication of application: **29.04.2026**
(21) Application number: 25209744.9
(22) Date of filing: 20.10.2025
(51) Int. Cl.: A61B 5/11, A61B 5/16, A61B 5/00, G16H 50/20

(54) **A SYSTEM FOR GENERATING AN ALERT SIGNAL**

(30) Priority: 25.10.2024 FI 20246263
(71) Applicant: IXI Eyewear Oy, 02150 Espoo (FI)
(72) Inventor: Lehmuskallio, Harri, 00100 Helsinki (FI); Virtanen, Juha, 00560 Helsinki (FI)
(74) Representative: Moosedog Oy

(57) **Abstract**

An aim of the present disclosure is to provide a system (100, 200) for generating a log (102, 202) related to an onset event. The system (100, 200) comprises an ambient light sensor (104, 204) (106, 206) to monitor a light flickering pattern (302-306) and an eye tracking sensor (108, 208) to monitor an eye response (402-406) of an eye (110, 210). The system (100, 200) comprises a computing unit (112, 214) to collect the light flickering pattern (302-306) from ambient light sensor (104, 204) and the eye response (402-406) from eye tracking sensor (108, 208) during a first time period. Computing unit (112, 214) receives at a first moment of time, first user feedback related to onset event, such as migraine episode (506). Computing unit (112, 214) utilizes collected light flickering pattern (302-306), collected eye responses (402-406) and received user feedback as a log (102, 202) related to onset event.

## Description

### TECHNICAL FIELD

The present disclosure generally relates to health event onset detection and particularly to a system for generating a log related to an onset event. The present disclosure further relates to a system for generating a log entry related to an onset event.

### BACKGROUND

Globally, neurological disorders such as migraines are among the most common health billion people worldwide. Such disorders, including neuro-ophthalmic conditions such as photophobia, oscillopsia and photosensitive seizures, are triggered by stimuli beyond individual control. For example, a migraine episode can be triggered by exposure to flickering lights, changes in light intensity or specific light frequencies. It will be appreciated that such stimuli may not be directly perceivable due to their position in the environment or because the frequency of the flickering light is beyond the conscious perception range of human eye. Consequently, individuals may remain oblivious to the presence of such triggers, making it challenging to avoid triggering of the migraine episode.

Further, stimuli that can trigger a migraine episode or related neuro-ophthalmic conditions vary significantly between individuals. Some common triggers include stress, hormonal fluctuations, lack of food, sleep disturbances, neck pain, certain odours, alcohol use and changes in weather. Additionally, premonitory symptoms such as yawning, mood changes, lethargy, light sensitivity, difficulties in focusing vision, restlessness and feelings of cold or warmth can appear several hours or days before onset of a migraine episode. Conventional systems generally fail to monitor the diverse range of stimuli and premonitory symptoms associated with the triggering of migraine episodes or related neuro-ophthalmic conditions. Consequently, such systems fail to provide effective support for recognizing or predicting potential episodes based on specific triggers or symptoms, thereby preventing proactive management of the disorders and restricting individuals from taking preventive measures to reduce frequency or severity of such episodes.

In light of the above discussion, there exists an urgent need for solutions enabling accurate monitoring and recognition of diverse stimuli and premonitory symptoms associated with the onset of migraine episodes and/or similar neuro-ophthalmic disorders.

### SUMMARY

An aim of the present disclosure is to provide a system and method for generating a log related to an onset event. The system and method enable accurate collection of ambient light flickering patterns and correlation of such patterns with physiological responses of the user such as eye movements as well as user feedback, thereby creating a comprehensive log of onset events such as migraine episodes, photophobia and other neuro-ophthalmic conditions, as defined in the appended independent claims to which reference is made. Such collection and correlation of light flickering patterns and physiological responses enable detailed recording of onset events, enabling identification and management of future health onset events for the user. Advantageous features are set out in the appended dependent claims.

Another aim of the present disclosure is to provide a system and method for generating a log entry related to an onset event as defined in appended independent claims. The system and method enables real-time monitoring of environmental light conditions and physiological responses of the user, such as eye movements and other biometric parameters. Thus, the system enables to accurately determine onset events and generate relevant log entries for such events, thereby assisting in identification of future health onset events and contributing to effective management of adverse physiological reactions for the user.

Throughout the description and claims of this specification, the words "comprise", "include", "have", and "contain" and variations of these words, for example "comprising" and "comprises", mean "including but not limited to", and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 shows a diagram of a system for generating a log related to an onset event, in accordance with an embodiment of the present disclosure;
FIG. 2 shows a diagram of a system for generating a log entry related to an onset event, in accordance with an embodiment of the present disclosure;
FIG. 3 shows a graph illustrating light flickering pattern over time, in accordance with an embodiment of the present disclosure;
FIG. 4 shows a graph illustrating eye response of a user with respect to light flickering (such as, the light flickering pattern of FIG. 3) over time, in accordance with an embodiment of the present disclosure;
FIG. 5 shows a graph illustrating the progression of migraine symptoms over time, in accordance with an embodiment of the present disclosure; and
FIG. 6 shows a bar graph illustrating the prevalence of typical symptoms associated with neurological or migraine-related events, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

A system for generating a log related to an onset event, the system comprising:
an ambient light sensor, arranged on an eyewear, to monitor a light flickering pattern;
an eye tracking sensor, arranged on the eyewear to monitor eye response of an eye of a user; and
a computing unit configured to:
   collect during a first period of time, the light flickering pattern, as a function of time, from the ambient light sensor and the eye response of the user, as a function of time, from the eye tracking sensor;
   receive, at a first moment of time, a first user feedback related to the onset event such as at least one of: a migraine episode, a photophobia event, an oscillopsia event, a photosensitive seizure event, a neuro-ophthalmic disorder, a photopsia event and a diplopia condition; and
   use the collected light flickering pattern, the eye responses and the received user feedback as a log related to the onset event.

In a first aspect, the present disclosure provides a system for generating a log related to an onset event. Herein, the use of the collected light flickering pattern, the eye responses and the received user feedback as the log enables the computing unit to generate a structured multi-modal record rather than a set of isolated sensor readings as in case of the prior art. Generating the log in this manner ensures temporal alignment of environmental stimuli, physiological reactions and subjective symptom reporting, thereby providing a coherent ground-truth dataset. The log thus forms a technical basis for retrospective analysis and further predictive modelling, unlike conventional systems that only provide real-time monitoring and alerting. Further, the integration of both non-perceptible light flickering patterns, such as high-frequency modulations beyond conscious perception, and subconscious ocular responses allows the system to identify hidden environmental triggers that the user may not recognise. A technical benefit of such correlation is the ability of the system to detect adverse onset events arising from imperceptible stimuli, thereby offering improved reliability in identifying environmental conditions contributing to neurological or neuro-ophthalmic disorders. For example, the log allows clinicians and the user to trace back which environmental light patterns and ocular responses corresponded to specific onset events. Additionally, the same log provides training input for adaptive algorithms and artificial intelligence engines, thereby extending the system beyond reactive detection and into proactive prediction of onset events. This dual applicability increases the technical effect of the system over the prior art approaches that are restricted to immediate response functions. A synchronized recording of the light flickering pattern, eye responses and user feedback in the log improves data integrity and reliability, as it reduces ambiguity between cause and effect by linking specific environmental changes to corresponding physiological and subjective responses. This structured alignment ensures a robust correlation that cannot be achieved by unstructured historical data collection.

The term "system" as used throughout the present disclosure refers to a set of electronic components configured to monitor ambient light patterns and eye responses of a user and generate a log corresponding to an onset event. The term "onset event" as used throughout the present disclosure relates to any condition impacting neurological or visual health, such as a migraine episode, a photophobia event, an oscillopsia event, a photosensitive seizure event, a neuro-ophthalmic disorder, a photopsia event or a diplopia condition. The terms "migraine", "migraine activity" and "migraine attack" encompass various phases and symptoms associated with a migraine event, including stages such as throbbing or pulsating headaches, nausea, vomiting as well as the various effects related to the migraine episode.

The system comprises an ambient light sensor arranged on an eyewear to monitor a light flickering pattern. The term "ambient light sensor" as used throughout the present disclosure refers to an optical component capable of detecting variations in ambient light, including but not limited to, light intensity, frequency and flickering patterns. The term "light flickering pattern" as used throughout the present disclosure refers to a repetitive variation in light intensity occurring at various frequencies, such as high frequencies between 500 and 7000 Hz or changes in intensity not reaching zero. For example, a light flickering pattern comprises cases where a bright light fluctuates at a frequency imperceptible to a user but affects visual health of the user. It will be appreciated that high-frequency flickering of light at 20 Hz for over 90 seconds is known to trigger neurological symptoms such as migraine episodes in susceptible individuals.

The ambient light sensor enables the system to accurately detect such light flickering patterns, thereby enabling the system to identify environmental conditions contributing to the onset of adverse health episodes. The term "eyewear" as used throughout the present disclosure refers to any wearable apparatus configured to be positioned on head of the user. Further, the eyewear is equipped with optical or electronic components for monitoring or processing visual or physiological data. For example, the eyewear comprises smart-glasses, virtual reality (VR) headsets, augmented reality (AR) headsets or similar wearable devices designed to incorporate sensors or tracking systems. For example, the light sensor comprises photodiodes that are sensitive to various wavelengths of light, including visible, infrared and ultraviolet light. It will be appreciated that as the ambient light collides with the photodiodes, the light sensor generates an electrical current proportional to the intensity of the emitted light. Consequently, the stronger the emitted light, the more current is produced. The light sensor distinguishes between different levels of brightness or illuminance to monitor fluctuations in light intensity and also detect changes in lighting patterns, such as flickering or gradual dimming. In addition, the light sensor(s) such as photodetectors can comprise a set of photodetectors equipped with different colour filters. This arrangement is beneficial since some migraineurs react to blue or reddish light more strongly than to green light as an example. The positioning of the ambient light sensor on the eyewear enables detection of light variations from multiple angles, providing comprehensive ambient light data even if the light source is outside a direct field of view of the user. Such a configuration allows effective capturing of changes in light flickering patterns that remain unnoticed by the user, thus enhancing detection of potential environmental triggers. The light sensor is orientated or positioned away from the eye of the user on the eyewear to enable detection of light variations from multiple directions, thereby enabling that light sensor to capture a broad range of ambient light data. Such placement of the light sensor enables the light sources positioned outside a direct line of sight of user to be monitored effectively. Further, as the light sensor is orientated or positioned away from the direct line of sight of the user, the light sensor identifies subtle changes in light flickering patterns that go unnoticed by the user. Alternatively, the ambient light sensor may have a narrow angle (such as 1, 2, 3, 4, 5, 6, 7, 8, 9, 10, 15 degrees) of reception. In this case, the ambient light sensor scans the environment when the orientation of the eyewear changes. This is a way it is possible to obtain a bit more detailed information from the environment, especially when combined with accelerometer data. For example, a refresh rate of a monitor and contrast between monitor and background. As another example combining the ambient light sensor with accelerometer data provides way to obtain information of environment from various directions.

The system further comprises an eye tracking sensor arranged on the eyewear to monitor an eye response of an eye of a user. The term "eye tracking sensor" as used throughout the present disclosure refers to an electronic component capable of monitoring and recording various ocular parameters, including but not limited to, angular speed of pupil movement, blink rate, gaze direction, pupil dilation and saccadic movements. The term "eye response" as used throughout the present disclosure refers to changes in ocular parameters caused by external stimuli, such as changes in light flickering patterns or sudden variations in ambient light conditions. For example, a rapid saccadic movement or increased blink rate indicates visual strain due to light flickering. For example, the eye tracking sensor is implemented as an infrared (IR) camera that allows continuous monitoring of the eye without distracting or impacting the vision of user. Such an eye tracking sensor implemented as the IR camera is an image sensor that monitors eye behaviour by capturing high-resolution images or video of the eye. The image sensor analyses subtle movements such as blink rates and pupil reactions to environmental triggers. Thus, the IR camera enables real-time monitoring of eye response to external stimuli, such as sudden changes in light conditions. According to another example the eye tracking sensor can be a combination of infrared (IR) transmitting leds and infrared receiving photodiodes. In such example a transmitting led(s) emits a light pulse(es) towards an eye and one or more respective photodiodes measure amount of light reflected from a surface of the eye. The amount of light received by each of the photodiodes corresponds to direction of gaze.

Optionally, the IR camera also tracks surface temperature of the eye and surrounding area thereof, thereby, providing physiological data that serves as a biomarker for potential onset events. It will be appreciated that changes in eye surface temperature are early warning signs of an impending onset event, such as a migraine episode, a photophobia event or other neurological conditions. The detection of eye temperature along with light flickering and ocular metrics (such as pupil dilation or blink rate) enable to improve efficiency of the system to assess the likelihood of an onset event. Thus, the utilization of the eye temperature as the biomarker enables to improve the sensitivity of the system in predicting onset events, allowing earlier detection and personalized, proactive interventions to prevent or delay symptoms.

The eye tracking sensor enables to accurately capture a wide range of ocular movements, thereby, allowing the system to monitor eye responses indicative of visual fatigue, visual adaptation or abnormal ocular behaviour associated with the onset event. Consequently, the eye tracking sensor enables the system to distinguish between normal eye movements and those triggered by harmful environmental stimuli, thereby providing an accurate correlation between eye responses and potential triggers.

The system further comprises a computing unit configured to receive and process data from the ambient light sensor and the eye tracking sensor. The term "computing unit" as used throughout the present disclosure refers to an electronic component capable of processing data collected from the ambient light and eye tracking sensors and storing the processed data in a structured manner. The computing unit is configured to collect during a first period of time, the light flickering pattern, as a function of time, from the ambient light sensor and the eye response of the user, as a function of time, from the eye tracking sensor. The term "first period of time" as used throughout the present disclosure refers to a predefined duration during which the system continuously monitors light and eye response parameters to identify a specific type of the onset event. For example, the light flickering pattern comprises both visible and imperceptible modulations in light intensity that are not consciously recognised by the user but still affect visual processing thereof. The eye response data comprises parameters such as blink frequency, pupil dilation, fixation duration and saccades, indicating the visual and neurological state of the user.

The computing unit simultaneously processes and analyses both the light flickering pattern and the eye response of the user, thereby establishing a relationship between ambient light variations and ocular responses. Such an established relationship enables identification of specific patterns linked to premonitory symptoms or adverse health conditions. Consequently, the computing unit enables the system to predict the onset of adverse health conditions, such as migraine episodes, thereby supporting proactive intervention by the user before symptoms associated with the onset become severe.

The computing unit is further configured to receive, at a first moment of time, a first user feedback related to the onset event such as at least one of: a migraine episode, a photophobia event, an oscillopsia event, a photosensitive seizure event, a neuro-ophthalmic disorder, a photopsia event and a diplopia condition. The term "user feedback" as used throughout the present disclosure refers to an input provided by the user indicating an experience or anticipation of an onset event. The first user feedback is associated with specific symptoms or conditions, including migraine episodes, photophobia and other neuro-ophthalmic disorders.

The feedback includes manual inputs, such as reporting a migraine episode through a mobile health management application or automatic inputs based on predefined conditions. For example, the user manually marks an undetected migraine episode using the mobile health management application (running on a computing device such as smartphone, laptop and the like) for tracking future occurrences of migraine episodes. Further, the user provides the user feedback through various mechanisms, including but not limited to, manual inputs such as logging episodes via smartphone or laptop application, voice commands or through wearable devices.

The incorporation of the user feedback enables to provide valuable contextual information to the computing unit in associating user-reported symptoms with collected data. Such an integration further enables refinement of the correlation between environmental triggers and physiological responses, thereby improving accuracy of the generated log. The user feedback enables the system to better understand specific health patterns of each individual to refine the correlation between reported symptoms and monitored light flickering patterns. Such a personalized input of the user feedback enhances the capability of the system to accurately identify the relationship between environmental stimuli and migraine episodes, thereby improving effectiveness of the system at predicting and managing such conditions. Thus, as the impact of light flickering on migraine episodes varies from individual to individual, incorporating the user feedback into the system enables high level of personalization.

Further, the computing unit is configured to use the collected light flickering pattern, the eye responses and the received user feedback as a log related to the onset event. The term "log" as used throughout the present disclosure refers to a record storing the temporal variations of light patterns, the corresponding eye responses and the received user feedback to enable analysis of correlation between environmental stimuli and the onset event. The generated log enables a detailed analysis of conditions that trigger the onset event and provides insights for identifying potential triggers or premonitory symptoms. The generated log also provides historical record related to past onset events, such that the historical record allows retrospective analysis of the events preceding a most recent onset event. Thus, the generation of the log enables the system to identify trends in environmental conditions and physiological responses, thereby improving understanding of factors contributing to the onset of adverse health conditions. Such a log also provides the user an actionable history of environmental and physiological conditions, facilitating the development of individualized strategies for managing onset events and enabling targeted and effective interventions.

Thus, the system enables collection of ambient light flickering patterns, eye responses and user feedback to generate a comprehensive log related to onset events experienced by the user. Such a log provides insight into how various light conditions and visual responses correlate with adverse health episodes, thereby assisting in effective management and mitigation thereof. Further, an ability of the system to integrate multiple data sources, analyse complex patterns and incorporate user-reported information enhances an effectiveness thereof in predicting future onset events, enabling the user to prevent occurrences thereof by limiting or avoiding exposure to the causal conditions, such as ambient light flickering patterns.

In an embodiment, the monitored eye response is selected from: an angular speed of pupil movement, a gaze direction, a blink rate, a pupil dilation, a saccades, a fixation duration, a visual fatigue indicator, an abduction event, an adduction event, a dextroversion event, a laevoversion event, a dextroelevation event, a dextrodepression event, a laevoelevation event, a laevodepression event, a convergence event and a divergence event. In this regard, monitoring of the aforesaid ocular parameters enables the computing unit to capture subtle visual fatigue indicators that precede an onset event. A technical benefit of this is that the system recognizes early-stage disruptions that are not consciously perceived by the user, thereby allowing earlier intervention as compared to conventional systems. Further, an ability to track complex eye movements such as abduction, adduction, dextroversion and convergence provides a comprehensive dataset within the log. This expanded range of eye responses improves correlation accuracy between environmental triggers and neurological effects, enabling the system to distinguish between normal visual adaptation and pathological responses. Such differentiation is technically advantageous for reducing false positives in onset event detection. Additionally, the inclusion of fixation duration and visual fatigue indicators allows the system to quantify sustained strain under flickering or unstable lighting. Beneficially, this allows for a reliable prediction of conditions such as migraines or photophobia episodes triggered by prolonged exposure, which conventional alerting systems do not capture.

The term "saccade" as used throughout the present disclosure refers to rapid movement of the eye between fixation points that indicates visual fatigue or neurological disturbances. The system detects saccades occurring frequently during high cognitive load or visual fatigue, such that the saccades indicate premonitory symptoms of migraine.

Optionally, the system monitors additional parameters such as blink rate and fixation duration. Further, an increased blink rate or shortened fixation duration indicate visual discomfort caused by ambient light flickering or intensity variations. For example, a faulty monitor blinking at a low rate causes an elevated blink rate. Such an elevated blink rate is a physiological response indicating early signs of visual strain and a potential trigger of a migraine episode. Such an ability of the system to monitor the additional parameters enables the system to capture real-time indications of visual strain, thereby enabling the system to predict the onset of conditions that escalate into more severe adverse health episodes (such as a migraine episode) if not promptly addressed. Consequently, a capability of the system to monitor a diverse range of eye responses allows the system to recognize abnormal patterns indicative of potential onset events. The monitoring of diverse eye responses enables the system to identify subtle changes in ocular behaviour that are not readily apparent to the user but are indicative of underlying neurological symptoms. Consequently, the comprehensive monitoring enables the system to understand ocular behaviours that serve as biomarkers for various neurological and neuro-ophthalmic conditions.

The computing unit analyses the data received from the eye tracking sensor, such as the image sensor that captures high-frame rate images or video, to denote displacement of the pupil over time to detect angular speed of pupil movement. The computing unit analyzes the received images to calculate the speed of pupil movement as a function of light flickering. Similarly, the control unit analyzes images to monitor the position of each pupil relative to a center of the corresponding eye.

The system continuously maps the position of pupil and corneal reflection to determine gaze of the user. The system determines the gaze to track shifts in gaze direction of the user for identifying visual strain when the user frequently shifts the gaze, especially under challenging lighting conditions. Moreover, the system determines shifts in the eye position to determine saccade-related parameters such as frequency and speed of saccades upon exposure to flickering lights. Further, the system employs the determined gaze or pupil position to determine fixation duration (i.e., time duration during which the gaze remains steadily focused on a single point or object without shifting), dextroversion (i.e., movement of both eyes to a right-hand side of the user), laevoversion (i.e., movement of both eyes to a left-hand side of the user), dextroelevation (i.e., movement of both eyes simultaneously upward and towards the right-hand side of the user), dextrodepression (i.e., movement of both eyes simultaneously downward and toward the right-hand side of the user), laevoelevation (i.e., movement of both eyes simultaneously upward and towards the left-hand side of the user), convergence (i.e., movement of both eyes inward towards nose of the user), divergence (i.e., movement of both eyes outward away from the nose of the user) and laevodepression (i.e., movement of both eyes simultaneously downward and towards the left-hand side of the user). The detection of movement of the eyes in response to light flickering enables the system to assess various ocular motor responses to determine onset of neurological disruption events that may precede migraine episodes. Further, the system monitors the various eye responses to identify subtle changes to enable early detection of potential health issues, allowing timely interventions to prevent escalation of symptoms to complete migraine episodes.

Further, the system measures the blink rate by detecting the occlusion of the pupil or cornea (the occlusion can be detected for example a camera or with a pair of infrared (IR) led and infrared photo diode. While using the LED and photo diode occlusion i.e. a blink can be observed as intensity of reflected light changes when eye lid is closed in compared to when it is open) within a specified time frame. It will be appreciated that an increase in the blink rate indicates visual discomfort or strain, such as, when triggered by flickering light sources. Additionally, the computing device analyzes the captured images to determine pupil dilation (i.e., changes in the pupil size) over time in response to varying light intensities. Moreover, monitoring the pupil dilation helps the system to identify patterns of visual strain, light sensitivity or premonitory migraine symptoms. The system utilizes image or signal processing techniques to determine the position of the eye and pupil based on received data. The data for this can be for example a captured image/video frame to track an exact location and movement of the pupil or the data can be based on reflections of light from pupil (when using LED and photodiodes). This allows system to monitor various ocular parameters, such as, gaze direction, eye movement and other critical indicators such as saccades or fixations. In general any gaze tracking means can be used.

For example, during prolonged work in front of a computer monitor, users experience decreased blink rates due to visual fatigue, escalating into migraine episodes or other visual disturbances in susceptible users. This is probably due to intensive focusing on the work. This might lead to drying of eyes. On the other hand dry eyes lead to excessive blinking on long run (or at least to runs of blinks every now and then, even though the total blink rate may stay low). Thus, an ability of the system to capture such variations in ocular parameters enables early detection of migraine episodes caused by such environmental or occupational factors. Such a capability allows the system to distinguish between normal eye behaviour and abnormal responses, thereby providing precise correlation between eye responses and external triggers and enhancing an overall sensitivity of the system to early visual and neurological symptoms.

In an embodiment, collected monitored light flickering pattern comprises at least one of: a frequency of flickering, an intensity variation, a modulation depth as function of time. The term "frequency of flickering" as used throughout the present disclosure refers to a measure of how often the intensity of light fluctuates over a specified period of time, such as in cycles per second (Hz). It will be appreciated that high-frequency flickers between 500 and 7000 Hz are not noticeable by a majority of population but still trigger neurological symptoms like migraines, photosensitive seizures or other adverse conditions such as oscillopsia and visual disturbances in susceptible users. The ability of the system to accurately monitor the frequency of flickering enables the system to capture harmful patterns that are not perceptible to the human eye but still impact visual and neurological health. Thus, the monitoring of the frequency of flickering allows the system to identify flicker rates that are known to trigger specific neurological symptoms in susceptible users, thereby, enabling users to reduce or prevent exposure to harmful lighting conditions. The system employs light sensors, such as photodiodes to continuously detect light levels emitted by a light source and subsequently, generate an electrical signal in response. The generated electrical signal is analyzed by the computing unit to determine time intervals between peaks and troughs (i.e., one complete cycle of light intensity fluctuation). Further, the computing unit also determines how many cycles occur within one second to calculate the frequency of flickering. The term "intensity variation" as used throughout the present disclosure refers to changes in brightness or amplitude of emitted light over time, such that the changes affect visual perception depending on magnitude and rate of the changes. For example, intensity variations caused by faulty or unstable light sources, such as a defective fluorescent bulb, contribute to visual strain or neurological discomfort. Further, sudden variations or rapid fluctuations in intensity disrupts visual processing and leads to adverse health effects, such as triggering of migraine episodes in susceptible users. Thus, a capability of the system to monitor the intensity variations in real-time allows early identification and classification of harmful light patterns, thereby enabling proactive management of sources contributing to photophobia or photosensitive epilepsy. The system calculates both magnitude (amplitude) of the intensity variation (i.e., how much the brightness changes) and the rate at which intensity variation is observed. For example, a sudden drop from full brightness to dimness represents a large amplitude change. Further, if such a drop is observed over a very short time (such as in milliseconds), the system detects a rapid intensity variation. The term "modulation depth" refers to the extent of the variation in intensity relative to the maximum possible intensity. It describes how strong or pronounced the flicker is. This parameter varies with time, meaning the degree of flickering can change over the period of observation.

For example, low-rate modulations in light flickering pattern from a malfunctioning overhead light causes a sensation of visual instability, triggering conditions such as photophobia or oscillopsia. The capturing of such intensity fluctuations by the system and correlation thereof with physiological responses enables the system to identify specific light sources that need to be addressed (such as, fixed or replaced) to prevent adverse reactions. Further, an ability of the system to monitor both the frequency of flickering and intensity variations as functions of time allows the system to recognize complex light patterns and a potential impact thereof on health of users. Consequently, such a capability enables the system to differentiate between various flickering sources and isolating conditions that are most harmful to visual and neurological wellbeing of users.

It will be appreciated that the monitoring of the frequency of flickering as part of the light flickering pattern allows the system to detect high-frequency modulations that are not consciously perceived but still trigger neurological disruptions. A technical effect of this is that the system identifies harmful environmental conditions that prior art systems fail to recognise. Further, by capturing intensity variations within the light flickering pattern, the computing unit can distinguish between gradual dimming and abrupt fluctuations. This distinction provides a technical advantage in correlating specific intensity changes with user discomfort, thereby reducing misclassification of benign lighting conditions as harmful triggers. Additionally, the inclusion of the modulation depth enables the system to quantify the strength of flickering relative to a maximum brightness. A technical benefit of this is that the system can prioritise and filter events based on severity, ensuring that only physiologically relevant light variations are logged. This improves both detection accuracy and efficiency of data storage.

In an embodiment, the system further comprises an artificial intelligence engine training module, wherein the collected light flickering pattern, the collected eye response of the user and the first user feedback are used to train an artificial intelligence engine to automatically determine a type of the onset event. The term "artificial intelligence engine" as used throughout the present disclosure refers to a software-based algorithmic module capable of processing and analysing complex datasets to identify patterns, correlations and predictive indicators linked to the onset event. The artificial intelligence engine training module utilizes a combination of supervised and unsupervised learning algorithms to analyse the light flickering patterns, physiological responses and user feedback to generate a data model capable of recognizing specific conditions, such as a migraine episode, photophobia event, photosensitive seizure, oscillopsia or other neuro-ophthalmic disorders.

For example, the system trains the artificial intelligence engine to identify a 20 Hz flicker rate combined with increased saccadic movements and user-reported photophobia to determine characteristics of an imminent migraine episode. Such training enables the artificial intelligence engine to recognize specific combinations of light patterns and eye responses associated with different types of onset events, thereby improving the capability of the system to differentiate between various neuro-ophthalmic conditions. The artificial intelligence engine training module analyses such inputs in a regular manner and updates the data model based on new user feedback and recorded physiological responses, allowing the system to learn and adapt to unique sensitivities of each user. The artificial intelligence engine identifies triggers based on historical data and alerts the user in advance, enabling early intervention and mitigation of the onset event. Further, continuous training of the artificial intelligence engine using updated light flickering patterns, eye responses and user feedback allows the system to refine the data models, thereby improving the capability of the system to detect premonitory symptoms with improved precision. Consequently, the system adapts over time to unique health profile and environmental conditions of the user, enabling effective management of neuro-ophthalmic disorders for specific sensitivities of the user and further enabling to reducing a likelihood of false alerts.

It will be appreciated that the use of the artificial intelligence engine training module allows the computing unit to continuously refine correlations between the light flickering pattern, the eye responses, and the first user feedback. A technical benefit of this is an adaptive system that improves accuracy with ongoing use, unlike static threshold-based methods in the prior art. Further, the training of the artificial intelligence engine with such multimodal data enables recognition of complex patterns that cannot be detected by rule-based systems. A technical effect is that the system can classify subtle onset events and distinguish between overlapping conditions such as photophobia and migraine episodes. Additionally, the incorporation of user-specific feedback into the training process provides personalisation of event classification. A technical benefit is that the artificial intelligence engine evolves to reflect individual sensitivities, thereby enhancing predictive performance for each individual user.

In an embodiment, the system comprises a microphone configured to monitor an ambient sound level and wherein the computing unit is configured to use the monitored ambient sound level with the monitored eye responses and the monitored light flickering pattern to recalibrate the determined onset event. The term "microphone" as used throughout the present disclosure refers to an electronic component capable of converting sound waves into electrical signals to measure an ambient sound level in the surrounding environment of the user. For example, a sudden increase in the ambient sound level, such as a loud alarm or machinery noise, influences visual responses of the user and alters the perceived intensity of flickering patterns. Such a sudden change causes heightened blink rates and shifts in gaze direction, resulting in an inaccurate determination of an onset event when only visual data is considered.

The use of the microphone for monitoring the ambient sound level enables the system to also consider external auditory factors that affect visual behaviour. The computing unit receives the monitored ambient sound data and correlates the monitored ambient sound data with the light flickering patterns, the eye tracking data and the user feedback. The system recalibrates the analysis of the onset event based on such monitored ambient sound data to improve the precision of onset event detection by the system. For example, a high-frequency flickering light in combination with a loud background noise, such as from machinery, causes visual instability and elevates blink rates. Consequently, the system analyses both sound and visual data, ensuring that the detected onset event is accurately attributed to a harmful flickering pattern rather than an unrelated auditory distraction.

The integration of the microphone into the system allows the system to differentiate between onset events triggered by visual stimuli alone and those influenced by external auditory factors. Such an operation reduces false detections by isolating the specific trigger conditions associated with the onset event. For example, the recalibration of visual responses based on the ambient sound level ensures that the system accurately identifies the visual triggers contributing to an event such as photophobia while eliminating auditory noise as a misleading factor. The combined analysis of ambient sound levels and light patterns improves a reliability of the system in detecting onset events under varied environmental conditions, thereby increasing user trust and effectiveness of the system in enabling the user to manage adverse health episodes.

It will be appreciated that the inclusion of the microphone to monitor the ambient sound level allows the computing unit to account for auditory influences on ocular responses. A technical benefit is a reduction of false detections caused by sudden noises that may otherwise be misinterpreted as light-induced onset events. Further, the recalibration of onset event determination using both sound and light parameters enables the system to isolate visual triggers with higher precision. This improves robustness of event detection in environments where multiple sensory stimuli interact. Additionally, combining ambient sound data with light flickering patterns and eye responses provides a more holistic environmental context within the log. A technical benefit is a significant reduction in noise-corrupted data, ensuring that only physiologically relevant triggers are linked to onset events.

In an embodiment, the computing unit is configured to receive a biophysiological parameter of the user to determine a correlation between the monitored light flickering pattern and the potential onset event, wherein the biophysiological parameter is selected from: a heart rate, a skin temperature, skin conductance level, Electroencephalogram (EEG) data, Electromyography (EMG) data, a head position data, a blood pressure, and a heart rate variability. A technical benefit of this is that it can be confirmed that an observed eye response is part of a systemic neurological reaction, thereby reducing false positives from incidental ocular movements. Further, inclusion of parameters such as heart rate variability or skin temperature enhances sensitivity to premonitory phases of onset events. A technical effect is that the system can predict adverse conditions earlier than systems relying on visual monitoring alone. Additionally, integration of the head position data and the EMG data enables the system to differentiate between discomfort caused by poor posture and that triggered by flickering light. A technical benefit is improved diagnostic precision, ensuring that only flicker-related stimuli are logged as contributors to onset events.

The term "biophysiological parameter" as used throughout the present disclosure refers to physiological data reflecting the physical state and responses of the user. For example, the biophysiological parameter comprises heart rate, skin temperature, skin conductance level, Electroencephalogram (EEG) data, Electromyography (EMG) data, head position data, blood pressure, and heart rate variability.

It will be appreciated that heart rate and heart rate variability are indicators of autonomic nervous system activity that is altered during the premonitory phase of a migraine episode. The system continuously monitors heart rate variability to detect patterns of increased sympathetic activity or reduced parasympathetic activity that are experienced during early migraine phases. For example, elevated heart rate variability combined with heightened blink rates and abnormal gaze direction signals the onset of a migraine episode triggered by external light conditions. The system further processes skin temperature and skin conductance levels to detect changes in blood flow and sweat gland activity, respectively, associated with increased stress levels or autonomic disturbances during an onset event.

The EEG data provides information related to electrical activity of the brain, such that the provided information indicates changes in brain states associated with hyperactivity or fatigue that are characteristic of neurological conditions such as migraines or seizures. For example, a sudden increase in beta wave activity detected through EEG along with specific light flickering patterns might correlate to a premonitory phase of a migraine episode. Further, the EMG data might be used to measure muscle activity that is relevant for detecting neck stiffness or tension that accompanies a migraine episode. Such a detection of the EMG data enables the system to evaluate muscle activity in the head and neck region to identify symptoms such as neck tension-related headaches or visual disturbances caused by abnormal head posture.

Moreover, the head or neck position data allows the system to analyse head movements and position changes that are indicative of visual fatigue or discomfort. The system utilizes an accelerometer or gyroscope (for example, an accelerometer or gyroscope associated with a wearable device) to track the head and/or neck movements and positional changes. For example, a change in head position during exposure to flickering light indicates that the user is subconsciously trying to avoid the harmful light stimulus, thereby signalling the onset of a migraine episode led by visual discomfort. Further, blood pressure is a parameter that provides real-time insights into a vascular state of the user. For example, an increase in blood pressure combined with other eye and light monitoring data is associated with elevated stress or premonitory phases of neuro-ophthalmic conditions.

Optionally, the system receives such biophysiological parameters through wearable sensors integrated within the eyewear or connected via external devices, such as wristbands or headbands. The computing unit correlates the received parameters with the monitored light flickering pattern to enhance the precision of onset event detection. For example, when the system detects a high-frequency flickering light along with elevated heart rate and increased skin conductance, the computing unit determines that a photophobia event or a stress-induced migraine episode is likely to occur. Such an integration of the biophysiological parameters enables the system to provide a comprehensive analysis of the onset event, ensuring that external factors and internal physiological responses are considered together for accurate event prediction and intervention.

A system for generating a log entry related to an onset event, the system comprising:
an ambient light sensor, arranged on an eyewear, to monitor a light flickering pattern;
an eye tracking sensor, arranged on the eyewear to monitor eye response of an eye of a user; and
a computing unit configured to:
   collect during a second period of time the monitored light flickering pattern from the ambient light sensor and the monitored eye response of the user from the eye tracking sensor;
   use the monitored eye response and the monitored light flickering pattern to determine the onset event that triggers, at a second moment of time, at least one of: a migraine episode, a photophobia event, an oscillopsia event, a photosensitive seizure event, a neuro-ophthalmic disorder, a photopsia event and a diplopia condition, wherein a type of the onset event is determined using an artificial intelligence engine, which has been trained using the collected light flickering pattern, the collected eye response of the user and a first user feedback collected during a first period of time, wherein the first user feedback is related to the onset event; and
   generate a log entry, if a triggering onset event is determined.

In a second aspect, the present disclosure provides a system for generating a log entry related to an onset event. Herein, an ability of the computing unit to determine a type of the onset event using the artificial intelligence engine provides a technical improvement over conventional threshold-based detection. By training the artificial intelligence engine on the collected light flickering pattern, the collected eye response and the first user feedback, the system recognises subtle correlations between environmental stimuli and physiological reactions that are not apparent to the human observer or simple rule-based algorithms. A technical benefit is that the system adapts to complex real-world conditions where onset events are triggered by combinations of factors rather than single parameters. Further, the generation of the log entry only when the triggering onset event is determined ensures that the log is populated with relevant and validated information. This selective logging prevents the accumulation of noise data and guarantees that the log represents actual event correlations with high integrity. A technical effect is a structured, high-quality dataset within the log that supports both immediate alerting and retrospective analysis, which is not achievable by systems that indiscriminately record sensor data without classification. Additionally, the determination of the type of the onset event by the artificial intelligence engine introduces personalisation into the system. Since the artificial intelligence engine is trained with user-specific feedback from the first period of time, the resulting log entries reflect individual sensitivities, such as a heightened reaction to particular flicker frequencies or colours. A technical advantage is that each log entry corresponds to the unique health profile of the user, thereby enhancing prediction accuracy and enabling targeted interventions. Moreover, the system provides multimodal verification that ensures determination of the onset event is not based on a single noisy measurement but on consistent patterns observed across multiple modalities. A technical benefit is a significant reduction in false alarms and improved trust in system outputs, which directly addresses limitations of prior art that rely on narrower data sources.

The system comprises an ambient light sensor arranged on the eyewear to monitor a light flickering pattern, and an eye tracking sensor arranged on the eyewear to monitor an eye response of an eye of a user. The system further comprises a computing unit configured to collect during a second period of time the monitored light flickering pattern from the ambient light sensor and the monitored eye response of the user from the eye tracking sensor. The term "second period of time" as used throughout the present disclosure refers to a predefined duration during which the system continuously monitors light and eye response parameters to identify a specific onset event type.

The computing unit is configured to use the monitored eye response and the monitored light flickering pattern to determine the onset event that triggers, at a second moment of time, at least one of: a migraine episode, a photophobia event, an oscillopsia event, a photosensitive seizure event, a neuro-ophthalmic disorder, a photopsia event and a diplopia condition. The computing unit automatically identifies the onset event type based on predefined criteria and correlation between the real-time monitored data. For example, the computing unit detects a 20 Hz flicker pattern along with increased saccadic frequency and abnormal fixation duration. The computing unit subsequently determines that the current conditions correspond to a photophobia event at the second moment of time.

The computing unit is further configured to generate a log entry, if a triggering onset event is determined. The computing unit further analyses the collected data over the second period of time to establish patterns and trends in the light flickering pattern and eye response. Subsequently, upon detection of a specific pattern such as repetitive abnormal pupil dilation in response to low-frequency light modulations, the computing unit determines that the onset event is a photosensitive seizure and generates the log entry corresponding to the photosensitive seizure. Such an ability of the system to autonomously determine the onset event type enables the system to generate the log entry at a second moment of time, even if the user is not actively experiencing the adverse health episode at that moment.

The system also allows the user to provide feedback for each log entry. For example, the user confirms whether the recorded event accurately reflects a personal experience (instantly or after specific duration) of the event such as a migraine episode, a photophobia event, an oscillopsia event, a photosensitive seizure event, a neuro-ophthalmic disorder, a photopsia event and a diplopia condition. The feedback allows the system to finetune predictive capabilities by learning from user-specific patterns and sensitivities. Such user feedback enables the system to better recognize and anticipate triggers over time, thereby providing a more personalized and accurate response to the user.

Moreover, the user feedback enables the system to determine whether a particular log entry correctly identifies trigger event along with the time duration between the logged event and the actual onset of symptoms. The user feedback allows the system to personalize determination sensitivity thereof for the user to environmental triggers, such as light flickering. Such user feedback received on an ongoing basis enables the system to better recognize and anticipate triggers over time, thereby providing a personalized, accurate and proactive response tailored to neurological and visual health needs of user.

For example, once the system detects environmental triggers such as harmful light flickering patterns or intensity variations and correlates the detected environmental triggers with the physiological responses of the user (such as, increased blink rate or pupil dilation), the system suggests courses of action to delay or prevent onset events such as migraine episodes or photosensitive seizures. The system provides recommendations such as adjusting the brightness or flicker rate of digital screens/light sources, modifying the environment of the user by reducing exposure to specific light sources, switching off specific light sources, using tinted/polarized glasses or lenses that reduce the impact of light flickering and intensity on the eyes of the user and the like. Moreover, the system prompts the user to move away from the problematic environment or suggest relocating to a more visually stable area with better lighting conditions. Optionally, the system directly controls the light sources. For example, if a specific light is emitting flickers or intensity fluctuations that could trigger a migraine episode for the user, the system automatically dims or switches off the light to prevent the onset of symptoms. Similarly, the system also adjusts the brightness or flicker rate of digital screens, such as computer monitors or TVs, to user-specific settings to reduce visual strain.

Further, the system triggers an alert to provide timely and actionable notification to help users to manage potential onset events before the symptoms escalate. For example, when the system detects that the user is approaching a risky threshold such as prolonged exposure to harmful light flickering or intensity fluctuations, the system triggers push notifications to the smartphone of the user about the detected risk and recommended actions. Further, the system provides a subtle vibration on the device, such as the smartwatch or smartphone of the user, as haptic alert or notification. Optionally, the system generates the alert to a designated set of caretakers or family members in the event of a high-risk situation, such as a seizure or severe migraine episode that could impact the long-term health of the user.

Notably, the onset event type is determined using the artificial intelligence engine, which has been trained with an artificial intelligence engine training module as described hereinbefore. The term "onset event type" as used throughout the present disclosure refers to a specific health condition, such as a migraine episode, a photophobia event, a photosensitive seizure event or a neuro-ophthalmic disorder and the like that is triggered by specific external and internal stimuli. The artificial intelligence engine employs historical data, physiological inputs and recorded light flickering patterns to accurately classify the onset event type. The artificial intelligence engine operates by comparing real-time monitored data from the ambient light sensor and the eye tracking sensor against stored patterns in a trained data model to identify the specific type of onset event.

For example, when a light flickering pattern with a frequency of 20 Hz is detected and the eye responses of the user show an elevated blink rate with an observed change in saccadic movement, the artificial intelligence engine classifies such a combination as an indicator of an impending migraine episode. The artificial intelligence engine continuously analyses the incoming data and updates predictions thereof based on evolving conditions and recorded user feedback. The artificial intelligence engine refines the detection of onset event types by learning from the incoming data, enabling the artificial intelligence engine to adapt analysis capabilities of the system to suit unique health profiles of different users.

Consequently, the integration of the artificial intelligence engine allows the system to identify adverse health conditions with improved accuracy, enhancing the capability of the system to differentiate between similar visual triggers and pinpointing specific premonitory patterns for various neuro-ophthalmic disorders. For example, the artificial intelligence engine enables the system to accurately distinguish between a photophobia event caused by ambient light flickering and a migraine episode triggered by sudden changes in light intensity.

In an embodiment, the second moment of time is during the second period of time and the second moment of time is used as log entry time for the onset event. Herein, the use of the second moment of time as the log entry time ensures that the log precisely reflects when the onset event is detected. A technical benefit is a time-synchronized record that allows retrospective correlation between external stimuli, physiological reactions and onset symptoms with improved accuracy. Further, alignment of log entry time with the second period of time provides consistency across recorded events. A technical effect is that each log entry becomes a reliable temporal marker for later analysis or for training predictive models, unlike prior systems that do not preserve precise event timestamps. Additionally, by recording the second moment of time as the log entry, the system enables users and clinicians to reconstruct the sequence of environmental and physiological changes leading to the onset event. This enhances traceability of causal factors, which improves both event prediction and long-term management strategies. The term "second moment of time" as used throughout the present disclosure refers to a specific point within the second period of time during which the computing unit detects a correlation between the monitored light flickering pattern and the recorded eye responses, indicating a specific type of onset event. Such second moment of time is used as a timestamp in the log entry to provide a precise temporal reference for documenting the onset of the detected adverse health episode.

For example, when a photosensitive seizure is triggered by a light flicker at 500 Hz, the system identifies the second moment of time as the exact point when the eye tracking data of the user indicates abnormal pupil dilation, and the ambient light sensor registers the high-frequency light flickering pattern. The computing unit records the moment as the log entry time, creating an accurate timeline of the onset event for retrospective analysis and future reference. Such recording of the second moment of time as the log entry time allows the system to maintain a detailed history of onset events, supporting precise correlation between external stimuli and physiological reactions over time. For example, if photosensitive seizure events are repeatedly logged under similar lighting conditions, the system identifies the triggering of the photosensitive seizure events and the lighting conditions as a recurring pattern, enabling the user to avoid environments with similar triggers. The timestamped log entries enable a structured approach to documenting of onset events by providing an organized record that helps to identify specific triggers and associated conditions.

Consequently, the accurate documenting of the onset event with a temporal marker allows the system to provide the user with an overview of recurring patterns, enabling retrospective evaluations and proactive management of conditions influenced by both external and internal factors. The log entries generated by the system support effective tracking and visualization of event progression, assisting the user in identifying unknown triggers and implementing strategies to prevent the occurrence of future episodes.

In an embodiment, the second moment of time is before the onset event is triggered and the artificial intelligence engine is used to record log entry to a third moment of time, which is after the second moment of time to provide future log entry. The term "third moment of time" as used throughout the present disclosure refers to a point in time following the second moment of time, indicating when the system predicts that the onset event is likely to happen based on detected patterns and predictive analysis. The computing unit analyses historical data, real-time monitored parameters and environmental conditions to anticipate the onset event and subsequently logs the third moment of time as a future entry within the generated log.

It will be appreciated that the ability of the computing unit to generate the future log entry at the third moment of time provides predictive functionality rather than reactive detection. A technical benefit is that the system anticipates onset events before they occur, allowing preventive action to be taken. Further, recording the third moment of time as part of the log creates a temporal link between predictive indicators and an actual occurrence of the onset event. A technical effect is creation of comprehensive event history that improves training of the artificial intelligence engine and strengthens its predictive accuracy over time. Additionally, by distinguishing between the second moment of time (detection) and the third moment of time (prediction), the system (provides both real-time and forward-looking information. A technical advantage is improved user trust and clinical utility, since the log does not merely document past conditions but also forecasts likely future events.

For example, when the system detects a sudden increase in high-frequency flicker intensity along with a significant rise in heart rate variability and skin conductance, the computing unit determines that such parameters indicate a high probability of a migraine episode in the near future. The computing unit identifies the third moment of time within the upcoming minutes or hours (depending on an intensity of the pattern) as the likely time when the migraine episode will occur. Such third moment of time is recorded as a future log entry, thereby marking a prediction point within the log.

Optionally, the eyewear comprises an accelerometer and gyroscope that monitors movement and physical activity or lack thereof, such as restlessness or lethargy. The analysis of such data from the accelerometer and gyroscope enables the system to assess whether the user is experiencing heightened restlessness, thereby indicating fatigue or the onset of a neurological condition. The physical movement data is correlated with ocular parameters such as blink rate, pupil dilation and saccadic eye movements, as well as environmental factors like light flickering patterns to determine possible onset of a migraine or neuro-ophthalmic condition.

The recording of the future log entry enables the system to provide predictive insights, allowing the user to take preventive measures before the actual onset of the adverse health condition. The system continuously updates the future log entry based on incoming data, improving prediction capabilities of the system in detecting such future onset events and ensuring that the recorded third moment remains relevant as new information is processed. Consequently, the recording of the future log entry allows the user to receive an accurate and dynamic indication of when the onset event is expected to occur, supporting timely implementation of preventive strategies to reduce exposure to potential triggers, take pre-emptive medication or alter their environment to mitigate the impact of the anticipated condition.

Such a capability enables the system to let users actively prevent adverse health conditions, enhancing the effectiveness of the system in managing neurological and visual health conditions. Thus, the system provides both real-time and forward-looking data to the user, supporting comprehensive management of health conditions and minimizing the risk of severe adverse health episodes.

In an embodiment, the future log entry is used to generate an alert for the user. The term "alert" as used throughout the present disclosure refers to a notification or indication transmitted by the system to inform the user about a detected or anticipated adverse condition. The computing unit generates the alert when the recorded future log entry indicates that a specific onset event is likely to occur at the third moment of time. The alert is transmitted in real-time, providing the user with relevant information regarding the predicted onset event and suggesting recommended actions to prevent a potential onset.

For example, if the system determines that a migraine episode is expected to occur within the next 30 minutes based on a sudden increase in heart rate and a high-frequency light flicker pattern, the system generates an alert to indicate that the user should reduce exposure to bright or flickering lights. Optionally, the alert also recommends the user to take preventive measures, such as administering medication or adjusting the environment to avoid triggering the predicted condition. The alert is transmitted through a connected device, such as a smartphone or wearable display, ensuring that the user is promptly informed about the predicted onset event.

The use of future log entries to generate alerts enables the system to provide timely and reliable notifications that are tailored to the specific health profile of the user. Such alerts are based on predictive analysis, ensuring that the user receives information before the onset. For example, the alerts include actionable recommendations to prevent the onset of a photophobia episode, such as by avoiding certain environments, changing light settings or taking breaks while working in front of a computer monitor for extended durations.

Consequently, the combination of predictive logging and real-time alerts allows the system to provide a comprehensive solution for both monitoring and managing adverse neurological and visual conditions, improving the ability of the user to proactively respond to potential triggers and maintain better control over their health. It will be appreciated that the use of the future log entry to generate an alert provides the user with advance warning before an onset event fully develops. A technical benefit is timely intervention, enabling the user to adjust environment, take medication or avoid exposure to harmful conditions. Further, the generation of the alert directly from a predictive log entry ensures that warnings are based on verified correlations between environmental stimuli and physiological responses. A technical effect is improved reliability of notifications compared to generic threshold-based alert systems. Additionally, linking alerts to predictive log entries creates a closed feedback loop in which user actions can be correlated with system predictions. A technical advantage is that the computing unit can refine future predictions based on user behaviour, thereby enhancing personalisation and long-term accuracy.

Optionally, the eyewear comprises a skin conductivity sensor arranged on temples or nose pads of the eyewear to monitor physiological responses by measuring changes in the ability of the skin of the user to conduct electrical currents. The skin conductivity sensor is mounted on areas of the eyewear (such as a virtual reality headset, a spectacle or digital eyewear) that remain in direct contact with the skin of the user, such as around the eyes, cheek, ear, nose pads or temple areas, for continuous skin conductivity measurement. Further, when the user is exposed to external stimuli, such as light flickering or other environmental stressors, the body responds by increasing sweat production, even in minimal amounts, thereby altering the skin conductivity and providing a measurable indicator of stress or arousal level of the user. The system analyses changes in skin conductivity as a key biomarker to determine the possible onset of events such as migraines. For example, a sudden rise in skin conductivity signals an increase in stress or visual strain triggered by environmental factors, alerting the system to a potential onset event. Thus, the integration of skin conductivity data along with other monitored parameters such as eye movement, temperature and light flickering patterns, enables the system to accurately predict and allow prevention of adverse health events such as migraine episodes.

According to one aspect a method for generating a log related to an onset event is provided, the method comprising:
monitoring a light flickering pattern;
monitoring eye response of an eye of a user;
collecting during a first period of time, the light flickering pattern, as a function of time
receiving, at a first moment of time, a first user feedback related to the onset event such as at least one of: a migraine episode, a photophobia event, an oscillopsia event, a photosensitive seizure event, a neuro-ophthalmic disorder, a photopsia event and a diplopia condition; and
using the collected light flickering pattern the eye responses and the received user feedback as the log related to the onset event.

The method ensures that the log is created from both sensor inputs and user feedback, providing a holistic correlation between external triggers and individual perception, which conventional methods, that omit user inputs, cannot achieve. Further, collection of the light flickering pattern and the eye response during the first period of time provides temporally aligned data. A technical effect is a synchronized dataset that enables precise cause-and-effect analysis between stimuli and physiological responses. Additionally, by structuring the method around the first moment of time when user feedback is received, the system aligns subjective symptom onset with measured environmental and ocular parameters. A technical advantage is improved diagnostic accuracy and personalization of onset event prediction.

According to further embodiment method comprises training an artificial intelligence engine training module using the collected light flickering pattern, the collected eye response of the user and the first user feedback to automatically determine a type of the onset event. This method provides efficient way to customize the events based on the user type as the collection is related to feedback of the user.

Above aspect can be implemented in a computer system or a system as discussed above. It will be appreciated that training the artificial intelligence engine with multimodal data enables the method to capture complex correlations between light flickering patterns, eye responses and user feedback. A technical benefit is improved recognition of onset event types beyond the capability of threshold-based approaches. Further, continuous refinement of the artificial intelligence engine through new training inputs ensures that the method adapts to changes in user sensitivity over time. Thus, a dynamic and personalised prediction model is obtained, rather than a static detection model. Additionally, incorporating the user feedback as a training parameter ensures that the artificial intelligence engine learns from both objective and subjective indicators. A technical advantage is reduction of false positives and improved classification accuracy for real-world onset events.

According to further aspect a method for generating a log entry related to an onset event, the method comprising:
monitoring a light flickering pattern;
monitoring eye response of an eye of a user;
collecting during a second period of time the monitored light flickering pattern and the monitored eye response of the user;
using the monitored eye response and the monitored light flickering pattern for determining the onset event that triggers, at a second moment of time, at least one of: a migraine episode, a photophobia event, an oscillopsia event, a photosensitive seizure event, a neuro-ophthalmic disorder, a photopsia event and a diplopia condition; and
generating the log entry if a triggering onset event is determined.

Herein, generating the log entry only when the triggering onset event is determined ensures that the method produces a curated dataset rather than a continuous stream of raw values. A technical benefit is reduced storage of irrelevant data whilst maintaining high-quality log entries records. Further, aligning the determination of the onset event with the second moment of time provides precise temporal tagging of adverse conditions. A technical effect is improved traceability and retrospective analysis, which strengthens both diagnosis and predictive modelling. Additionally, by explicitly correlating the monitored light flickering pattern with eye responses at the second moment of time, the method captures causality rather than mere correlation. A technical advantage is enhanced reliability in linking environmental triggers to specific onset events, which prior art systems that only store raw histories cannot achieve.

According to an embodiment the method further comprises using an artificial intelligence engine for determining the onset event type. The artificial intelligence engine has been trained with an artificial intelligence engine training module as described above. According to method the determination is done using trained artificial intelligence engine. The training of the artificial intelligence engine is, according to an embodiment, carried out using previously collected patterns and responses combined with user feedback. Technical benefit of this is that it is possible to collect automatically log related to onset events and further to provide pre warnings to the user on likely future onset event. It will be appreciated that the determination of a type of the onset event using the artificial intelligence engine ensures that the method can provide event classification rather than generic detection. A technical benefit is that the log entry captures not only that the onset event occurred but also its precise type, such as migraine episode or photophobia event. Further, the training of the artificial intelligence engine with the multimodal data from light flickering patterns, eye responses and user feedback allows differentiation between similar conditions. A technical effect is reduced misclassification where conventional systems might conflate distinct onset events. Additionally, the ability to determine onset event types enhances the utility of the log for retrospective and clinical analysis. This facilitates clinicians and users to gain actionable insights into which categories of events are most frequently triggered, thereby enabling targeted interventions.

### DETAILED DESCRIPTION OF DRAWINGS

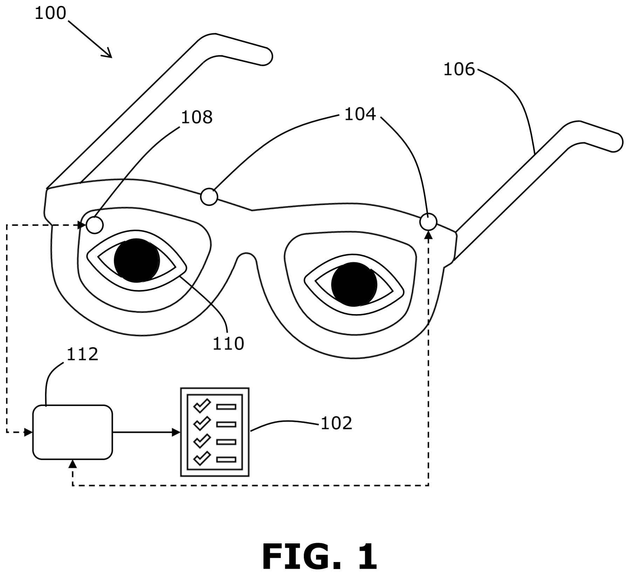

Referring to FIG. 1, there is shown a diagram of a system **100** for generating a log **102** related to an onset event, in accordance with an embodiment of the present disclosure. The system **100** comprises an ambient light sensor **104** arranged on an eyewear **106** to monitor a light flickering pattern. The system **100** further comprises an eye tracking sensor **108** arranged on the eyewear **106** to monitor eye response of an eye **110** of a user. Moreover, the system **100** comprises a computing unit **112** configured to collect during a first period of time, the light flickering pattern, as a function of time, from the ambient light sensor **104** and the eye response of the user, as a function of time, from the eye tracking sensor **108.** The computing unit **112** is further configured to receive, at a first moment of time, a first user feedback related to the onset event such as at least one of: a migraine episode, a photophobia event, an oscillopsia event, a photosensitive seizure event, a neuro-ophthalmic disorder, a photopsia event and a diplopia condition. Moreover, the computing unit **112** is configured to use the collected light flickering pattern, the eye responses and the received user feedback as the log **102** related to the onset event.

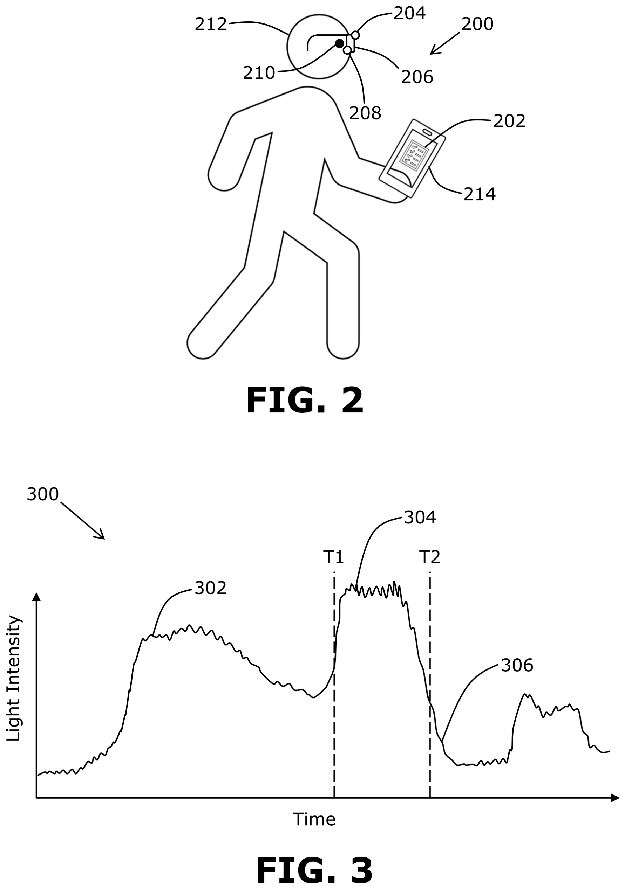

Referring to FIG. 2, there is shown a diagram of a system **200** for generating a log entry **202** related to an onset event, in accordance with an embodiment of the present disclosure. The system **200** comprises an ambient light sensor **204** arranged on an eyewear **206** to monitor a light flickering pattern and an eye tracking sensor **208** arranged on the eyewear **206** to monitor eye response of an eye **210** of a user **212.** The system **200** further comprises a computing unit **214** configured to collect during a second period of time the monitored light flickering pattern from the ambient light sensor **204** and the monitored eye response of the user **212** from the eye tracking sensor **208.** The computing unit **214** is further configured to use the monitored eye response and the monitored light flickering pattern to determine the onset event that triggers, at a second moment of time, at least one of: a migraine episode, a photophobia event, an oscillopsia event, a photosensitive seizure event, a neuro-ophthalmic disorder, a photopsia event and a diplopia condition, wherein a type of the onset event is determined using an artificial intelligence engine, which has been trained using the collected light flickering pattern, the collected eye response of the user **212** and a first user feedback collected during a first period of time, wherein the first user feedback is related to the onset event, and then generate the log entry **202,** if a triggering onset event is determined.

Referring to FIG. 3, there is shown a graph **300** illustrating light flickering pattern **302, 304, 306** over time, in accordance with an embodiment of the present disclosure. As shown, the light flickering pattern **302** varies in intensity over time, gradually increasing and decreasing in no discernible pattern prior to time T1. However, the light flickering pattern **304** between time T1 and T2 significantly intensifies, before gradually reducing to the indiscernible light flickering pattern **306** after time T2, which is similar to the light flickering pattern **302.**

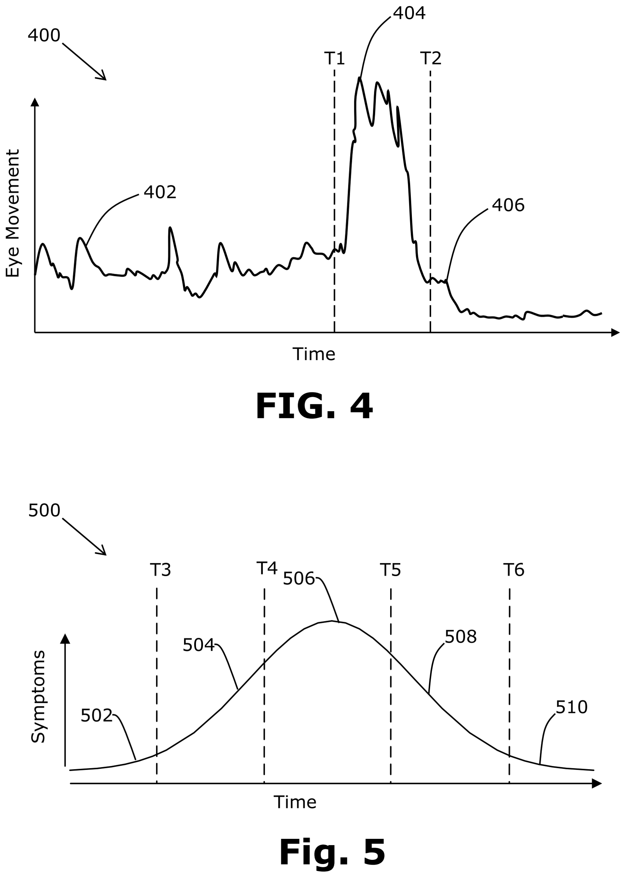

Referring to FIG. 4, there is shown a graph **400** illustrating eye response **402, 404, 406** of a user with respect to light flickering (such as, the light flickering pattern **302, 304, 306** of FIG. 3) over time, in accordance with an embodiment of the present disclosure. As shown, the eye movement **402** of the user prior to time T1 remains relatively stable. However, the eye movement **404** of the user increases drastically between time T1 and T2 corresponding to the increased intensity of the light flickering pattern **304** between time T1 and T2, potentially indicating an onset event, such as one of a migraine episode, a photophobia event, an oscillopsia event, a photosensitive seizure event, a neuro-ophthalmic disorder, a photopsia event or a diplopia condition. The eye movement **406** of the user reduces significantly after time T2 in response to reduction in the intensity of the light flickering pattern **306** (shown in Fig. 3) potentially indicating re-stabilization of the user post triggering of the onset event or suffering of an adverse reaction such as loss of consciousness.

Referring to FIG. 5, there is shown a graph **500** illustrating progression of migraine symptoms over time, in accordance with an embodiment of the present disclosure. As shown, graph **500** is divided into different phases **502, 504, 506, 508, 510,** such that each phase represents various stages of a migraine episode. The interictal migraine phase **502** occurs before time T3, where symptoms are minimal or absent. The phase **502** before T3 represents the period between migraine attacks when the user experiences little to no discomfort. The exemplary range for T3 can be 2 to 48 hours before migraine attack. At time T3, the preictal migraine phase **504** begins, characterized by the onset of gradual premonitory symptoms (such as irritability, fatigue or sensitivity to light). The preictal migraine phase **504** often lasts for hours to days, depending on the individual. At time T4, the ictal migraine **506** or migraine attack phase starts and symptoms reach peak intensity. The ictal migraine **506** between time T4 and T5 is most severe and can last between 4 to 72 hours, during which the user experiences debilitating symptoms such as intense headaches, nausea and heightened sensitivity to light and/or sound. Further, by time T5, the user enters the postictal migraine **508** or postdrome phase. The symptoms begin to decrease after time T5, but the user may still feel residual effects such as fatigue, confusion or soreness. Phase **510** between T5 and T6 lasts for several hours to days, with the symptoms gradually subsiding. Moreover, upon reaching time T6, the user enters a recovery state and symptoms for the user would have returned to a baseline level.

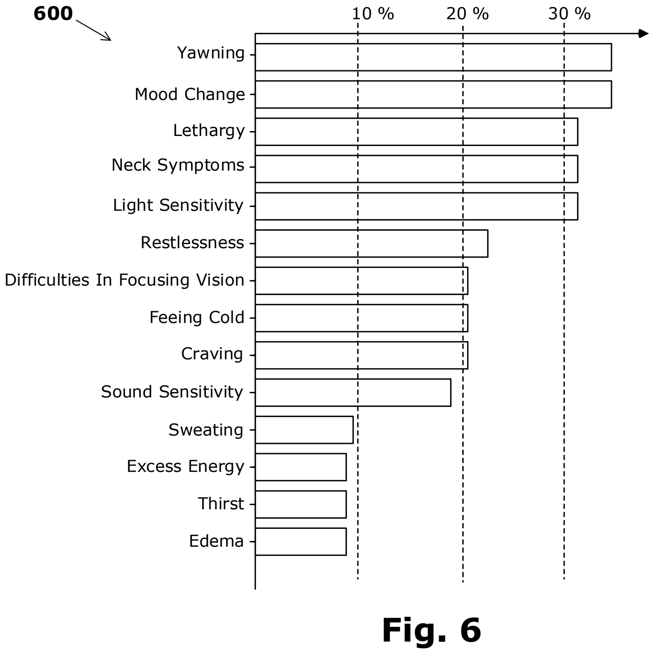

Referring to FIG. 6, there is shown a bar graph **600** illustrating the prevalence of typical symptoms associated with neurological or migraine-related events. The graph **600** lists symptoms such as yawning, mood changes, lethargy, neck symptoms and light sensitivity, among others, alongside their respective occurrence percentages. The eyewear **106** (shown in Fig. 1) is used to detect light flickering that induces onset of neurological or migraine-related events.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A system (100, 200) for generating a log (102, 202) related to an onset event, the system (100, 200) comprising:
an ambient light sensor (104, 204), arranged on an eyewear (106, 206), to monitor a light flickering pattern (302-306);
an eye tracking sensor (108, 208), arranged on the eyewear (106, 206) to monitor eye response (402-406) of an eye (110, 210) of a user (212); and
a computing unit (112, 214) configured to:
collect during a first period of time, the light flickering pattern, (302-306) as a function of time, from the ambient light sensor (104, 204) and the eye response (402-406) of the user (212), as a function of time, from the eye tracking sensor (108, 208);
receive, at a first moment of time, a first user feedback related to the onset event such as at least one of: a migraine episode (506), a photophobia event, an oscillopsia event, a photosensitive seizure event, a neuro-ophthalmic disorder, a photopsia event and a diplopia condition; and
use the collected light flickering pattern (302-306), the eye responses (402-406) and the received user feedback as the log (102, 202) related to the onset event.

2. The system (100, 200) according to claim 1, wherein the monitored eye response (402-406) is selected from: an angular speed of pupil movement, a gaze direction, a blink rate, a pupil dilation, a saccades, a fixation duration, a visual fatigue indicator, an abduction event, an adduction event, a dextroversion event, a laevoversion event, a dextroelevation event, a dextrodepression event, a laevoelevation event, a laevodepression event, a convergence event and a divergence event.

3. The system (100, 200) according to any of the preceding claims, wherein the collected monitored light flickering pattern (302-306) comprises at least one of: a frequency of flickering, an intensity variation, a modulation depth as function of time.

4. The system (100, 200) according to any of the preceding claims further comprising an artificial intelligence engine training module, wherein the collected light flickering pattern (302-306), the collected eye response (402-406) of the user (212) and the first user feedback are used to train an artificial intelligence engine to automatically determine a type of the onset event.

5. The system (100, 200) according to any of the preceding claims, wherein the system comprises a microphone configured to monitor an ambient sound level and wherein the computing unit (112, 214) is configured to use the monitored ambient sound level with the monitored eye responses (402-406) and the monitored light flickering pattern (302-306) to recalibrate the determined onset event.

6. The system (100, 200) according to any of the preceding claims, wherein the computing unit (112, 214) is configured to receive a biophysiological parameter of the user (212) to determine a correlation between the monitored light flickering pattern (302-306) and the potential onset event, wherein the biophysiological parameter is selected from: a heart rate, a skin temperature, skin conductance level, Electroencephalogram (EEG) data, Electromyography (EMG) data, a head position data, a blood pressure, and a heart rate variability.

7. A system (100, 200) for generating a log entry (102, 202) related to an onset event, the system (100, 200) comprising:
an ambient light sensor (104, 204), arranged on an eyewear (106, 206), to monitor a light flickering pattern (302-306);
an eye tracking sensor (108, 208), arranged on the eyewear (106, 206) to monitor eye response (402-406) of an eye (110, 210) of a user (212); and
a computing unit (112, 214) configured to:
collect during a second period of time the monitored light flickering pattern (302-306) from the ambient light sensor (104, 204) and the monitored eye response (402-406) of the user (212) from the eye tracking sensor (108, 208);
use the monitored eye response (402-406) and the monitored light flickering pattern (302-306) to determine the onset event that triggers, at a second moment of time, at least one of: a migraine episode (506), a photophobia event,
an oscillopsia event, a photosensitive seizure event, a neuro-ophthalmic disorder, a photopsia event and a diplopia condition, wherein a type of the onset event is determined using an artificial intelligence engine, which has been trained using the collected light flickering pattern (302-306), the collected eye response (402-406) of the user (212) and a first user feedback collected during a first period of time, wherein
the first user feedback is related to the onset event; and
generate the log entry (102, 202), if a triggering onset event is determined.

8. The system (100, 200) according to claim 6-7, wherein the second moment of time is during the second period of time and the second moment of time is used as log entry time for the onset event.

9. The system (100, 200) according to claim 7-8, wherein the second moment of time is before the onset event is triggered, and the artificial intelligence engine is used to record log entry (102, 202) to a third moment of time, which is after the second moment of time to provide future log entry (102, 202).

10. The system (100, 200) according to claim 9, wherein the future log entry (102, 202) is used to generate an alert for the user (212).

11. A method for generating a log (102, 202) related to an onset event, the method comprising:
monitoring a light flickering pattern (302-306);
monitoring eye response (402-406) of an eye (110, 210) of a user (212);
collecting during a first period of time, the light flickering pattern, (302-306) as a function of time
receiving, at a first moment of time, a first user feedback related to the onset event such as at least one of: a migraine episode (506), a photophobia event, an oscillopsia event, a photosensitive seizure event, a neuro-ophthalmic disorder, a photopsia event and a diplopia condition; and
using the collected light flickering pattern (302-306), the eye responses (402-406) and the received user feedback as the log (102, 202) related to the onset event.

12. The method (100, 200) according claim 11, further comprising training an artificial intelligence engine training module using the collected light flickering pattern (302-306), the collected eye response (402-406) of the user (212) and the first user feedback to automatically determine a type of the onset event.

13. A method (100, 200) for generating a log entry (102, 202) related to an onset event, the method (100, 200) comprising:
monitoring a light flickering pattern (302-306);
monitoring eye response (402-406) of an eye (110, 210) of a user (212);
collecting during a second period of time the monitored light flickering pattern (302-306) and the monitored eye response (402-406) of the user (212);
using the monitored eye response (402-406) and the monitored light flickering pattern (302-306) for determining the onset event that triggers, at a second moment of time, at least one of: a migraine episode (506), a photophobia event, an oscillopsia event, a photosensitive seizure event, a neuro-ophthalmic disorder, a photopsia event and a diplopia condition; and
generating the log entry (102, 202), if a triggering onset event is determined.

14. The method (100, 200) according to claim 13, wherein a type of the onset event is determined using an artificial intelligence engine, which has been trained with an artificial intelligence engine training module according to any of the claims 11-12.
